# EUROPEAN PATENT SPECIFICATION

(11) **EP 4 390 844 B1**
(45) Date of publication and mention of the grant of the patent: **08.04.2026**
(21) Application number: 23215934.3
(22) Date of filing: 12.12.2023
(51) Int. Cl.: G06T 7/11

(54) **OPHTHALMOLOGICAL DEVICE AND METHOD FOR CHARACTERIZING OPTICAL INHOMOGENEITIES IN AN EYE**
OPHTHALMOLOGISCHE VORRICHTUNG UND VERFAHREN ZUR CHARAKTERISIERUNG OPTISCHER INHOMOGENITÄTEN IN EINEM AUGE
DISPOSITIF OPHTALMOLOGIQUE ET PROCÉDÉ DE CARACTÉRISATION D'INHOMOGÉNÉITÉS OPTIQUES DANS UN IL

(30) Priority: 23.12.2022 CH 15852022
(43) Date of publication of application: 26.06.2024
(73) Proprietor: Ziemer Ophthalmic Systems AG, 2562 Port (CH)
(72) Inventor: ASSHAUER, Thomas, 5000 Aarau (CH); RATHJEN, Christian, 28197 Bremen (DE); ROTHEN, Franziska, 3011 Bern (CH); STEINLECHNER, Michael, 8006 Zürich (CH)
(74) Representative: Rentsch Partner AG

(56) References cited:
- SHI CE ET AL: "Machine learning helps improve diagnostic ability of subclinical keratoconus using Scheimpflug and OCT imaging modalities", vol. 7, no. 1, 10 September 2020 (2020-09-10), XP093030167, Retrieved from the Internet <URL:https://link.springer.com/article/10.1186/s40662-020-00213-3/fulltext.html> DOI: 10.1186/s40662-020-00213-3
- ZHANG XIAO-QING ET AL: "Machine Learning for Cataract Classification/Grading on Ophthalmic Imaging Modalities: A Survey", MACHINE INTELLIGENCE RESEARCH, SPRINGER BERLIN HEIDELBERG, BERLIN/HEIDELBERG, vol. 19, no. 3, 28 May 2022 (2022-05-28), pages 184 - 208, XP037870961, ISSN: 2731-538X, [retrieved on 20220528], DOI: 10.1007/S11633-022-1329-0
- SON KI YOUNG ET AL: "Deep Learning-Based Cataract Detection and Grading from Slit-Lamp and Retro-Illumination Photographs", vol. 2, no. 2, 2 June 2022 (2022-06-02), pages 100147, XP093029857, ISSN: 2666-9145, Retrieved from the Internet <URL:https://www.sciencedirect.com/science/article/pii/S2666914522000367/pdfft?md5=b1418b95b31a7ab1ea9756ddeec5a9f5&pid=1-s2.0-S2666914522000367-main.pdf> DOI: 10.1016/j.xops.2022.100147
- WAN ZAKI WAN MIMI DIYANA ET AL: "Towards a Connected Mobile Cataract Screening System: A Future Approach", JOURNAL OF IMAGING, vol. 8, no. 2, 10 February 2022 (2022-02-10), pages 41, XP093147315, ISSN: 2313-433X, DOI: 10.3390/jimaging8020041
- LI XIAORAN ET AL: "Simultaneous optical coherence tomography and Scheimpflug imaging using the same incident light", OPTICS EXPRESS, vol. 28, no. 26, 16 December 2020 (2020-12-16), pages 39660, XP093030154, DOI: 10.1364/OE.405643
- ZHUO ZHANG ET AL: "A survey on computer aided diagnosis for ocular diseases", BMC MEDICAL INFORMATICS AND DECISION MAKING VOLUME, BIOMED CENTRAL, LONDON, GB, vol. 14, no. 1, 31 August 2014 (2014-08-31), pages 80, XP021197123, ISSN: 1472-6947, DOI: 10.1186/1472-6947-14-80
- Z�BOULON PIERRE ET AL: "Development and validation of a pixel wise deep learning model to detect cataract on swept-source optical coherence tomography images", vol. 15, 10 October 2022 (2022-10-10), AMSTERDAM, NL, pages S43 - S49, XP093029847, ISSN: 1888-4296, Retrieved from the Internet <URL:https://www.sciencedirect.com/science/article/pii/S1888429622000553/pdfft?md5=905653defb5ff217350b4db2761ee637&pid=1-s2.0-S1888429622000553-main.pdf> DOI: 10.1016/j.optom.2022.08.003

## Description

### FIELD OF THE DISCLOSURE

The present disclosure relates to an ophthalmological device for characterizing optical inhomogeneities in an eye.

### BACKGROUND OF THE DISCLOSURE

Optical inhomogeneities in eyes are due to changes of the refractive index and can impair vision. Optical inhomogeneities can be caused by infection, injury, or congenital disorders, however most commonly due to age related decline. Optical inhomogeneities include cataracts, floaters, and opacification of the cornea. Corneal opacity occurs when the cornea becomes scarred. This stops light from passing through the cornea to the retina and may cause the cornea to appear white or clouded over. Common causes include infection, injury, corneal abrasion or scratch or swelling of the eye. Floaters in the eye appear as spots in vision, and is due to changes in the vitreous.

A cataract is a common, typically age-related eye disease characterized by progressive clouding of the lens. For treatment, the clouded lens is removed and replaced with a custom-fitted artificial lens. Because a large percentage of the world's population is affected by cataracts during their lifetime, cataract surgery is one of, if not the most commonly performed surgical procedure worldwide.

Typically, Phacoemulsification, or short "phaco", is a method of cataract surgery in which the lens is emulsified using ultrasonic energy for easier removal by aspiration with instruments inserted into the eye and attached vacuum pumping devices. Additionally or alternatively, lasers can be used to make the necessary minimally invasive access incisions in the eye tissue and to split the lens into smaller pieces for easier removal by aspiration.

The cataracts can be graded and classified using different systems. For example, the Lens Opacities Classification System (LOCS III) was introduced to classify the different cataract types and their severity using slit lamp and retroillumination images. Accurate knowledge of the severity of the cataract helps the ophthalmologist to optimally adjust his surgical technique. However, reproducible assessment according to LOCS III requires special training and additional labor-intensive steps for diagnostic screening.

Modern ophthalmological diagnostic systems, used prior to surgery as an aid for the ophthalmologist to make a diagnosis, record color photographs from a frontal view of the eye, as well as Scheimpflug images, and may include equipment for performing optical coherence tomography (OCT) to see details of the interior spaces of the eye.

Additionally, optical coherence tomography may be used immediately prior to surgery. The OCT images of the anterior chamber of the eye being used to position the laser incisions.

Optical coherence tomography provides a method for creating an optical cross-section of the eye which is non-invasive and high-resolution. The type of light used in OCT allows it to penetrate deep into tissue and examine tissue and fluid layers even with a high reflectivity. In OCT, typically many one-dimensional scans (A-scans) are performed at several positions (in particular lateral positions) along a pre-determined path in the eye. These A-scans can then be combined to form two-dimensional cross-sections of the eye (or parts thereof) in so-called B-scans. The B-scans can then be combined to form a three-dimensional image of the eye, also called a C-scan. With advanced OCT methods such as spectral domain OCT (SD-OCT), the acquisition time of OCT images has been shortened, and along with techniques to track and compensate for the movement of the eye and blinking, the accuracy of the OCT images has also improved.

DE102015009641A1 discloses a method for classifying a cataract of an eye for determining parameters for pre-setting phaco-treatment machines. In the method according to the invention for classifying a cataract of an eye for determining parameters for pre-setting phaco-treatment machines, OCT-based measurements are realized, the OCT-based scans are analysed using imaging technology and the local distribution of the cataract is determined, the cataract is classified on the basis of comparison values and the local distribution and classification of the cataract are used to ascertain parameters for pre-setting phaco-treatment machines. According to LOCSIII, however, cataract grade should be determined from slit lamp and retroillumination images, and therefore it is unclear whether OCT images alone contain sufficient information for grading cataracts.

US8360577B2 discloses a method for grading a cataract, the method including creating a three-dimensional image of at least most of a lens of an eye having a cataract from information received from an imaging system, comparing the three-dimensional image with a first template to determine an optical density of the lens, and determining a grade for the cataract based on the comparison of the optical density of the lens and a volume of the first template.

US10709610B2 discloses systems and methods for performing laser cataract surgery, for using a biometric system, which uses optical images of the eye to determine a material property of a structure of the eye, laser pulses in a laser shot pattern having different powers. This publication further discloses to determine, using only a common slit lamp imaging system to, e.g., grade the degree of a cataract, determine the relative location within the lens of different levels of opacifications, determine the relative location within the lens of different levels of increased density, e.g., different levels of hardness, compaction, toughness of the natural lens, increased density, and compaction, and provide a laser beam of varying power, with the power being predetermined to correspond to the degree of increased density, e.g., a predetermined shot pattern having specific, and varied in a predetermined manner, laser powers that correspond to the determined density, grade or other material properties of the lens. This publication does not mention high resolution data available from OCT imaging, for example, to accurately locate cataracts or classify the cataract grade. Nor does this publication include the detection of more general types of inhomogeneities of the eye other than cataract-like structures of the lens.

The publication Shi, Ce, et al. "Machine learning helps improve diagnostic ability of sub-clinical keratoconus using Scheimpflug and OCT imaging modalities." Eye and Vision 7 (2020): 1-12 discloses an automatic classification system which uses machine learning classifiers to distinguish between eyes with a keratoconus and a normal control population. This is enabled using Scheimplug images and ultra-high-resolution optical coherence tomography (UHR-OCT) images.

### SUMMARY OF THE DISCLOSURE

It is an object of the invention and embodiments disclosed herein to provide an ophthalmological device for characterizing optical inhomogeneities in an eye.

In particular, it is an object of the invention and embodiments disclosed herein to provide an ophthalmological device and method for cataract classification which does not have at least some of the disadvantages of the prior art, in particular in that it is more robust and has a higher detection confidence due to combining multiple complementary sources of data.

The present disclosure relates to a computer-implemented method for characterizing an optical inhomogeneity in a human eye. The method comprises receiving optical coherence tomography data of the eye. The method comprises receiving image data of at least one image of the eye recorded by a camera. The image(s) is recorded using the following imaging techniques: direct illumination of the eye, retroillumination of the eye, and/or Scheimpflug imaging. The method comprises characterizing the optical inhomogeneity as at least one of the following optical inhomogeneity types: a cataract, a floater, and/or an opacification of the cornea using the optical coherence tomography data and the image data.

In an embodiment, the camera is configured to record within the visible range of light. Optionally, the camera is configured to record a hyperspectral image, in particular in the infrared range up to 1350 nm.

In an embodiment, characterizing the optical inhomogeneity type further includes characterizing a cataract grade of the cataract.

In an embodiment, the cataract grade includes a cataract type comprising a nuclear sclerotic cataract, a cortical spoking cataract, and/or a posterior subcapsular cataract. The cataract grade may include a cataract severity level associated with at least one of the one or more cataract types. The cataract severity level may also be a general cataract severity level for all of the one or more characterized cataract types.

In an embodiment, the optical coherence tomography data includes one or more B-scans. Preferably, the optical coherence tomography data includes two or more B-scans arranged plane parallel to each other. Additionally or alternatively, the optical coherence tomography data includes two or more B-scans rotated about a common axis that is substantially parallel to an optical axis of the eye or the optical coherence tomography system.

In an embodiment, characterizing the optical inhomogeneity types comprises identifying one or more area segments of opacity in the eye using the optical coherence tomography data and/or the image data. Preferably, at least one of the area segments of opacity in the eye includes, or has associated therewith, a location in the eye, a two-dimensional size, a two-dimensional shape, a degree of opacity, and/or a distribution of opacity. The method includes characterizing the optical inhomogeneity using the one or more area segments.

In an embodiment, characterizing the optical inhomogeneity types comprises generating a three-dimensional representation of at least part of the eye using the optical coherence tomography data and/or the image data. The method comprises identifying one or more volume segments of opacity in the eye using the three-dimensional representation. Preferably, at least one volume segment includes, or have associated therewith, a location in the eye, a three-dimensional size, a three-dimensional shape, a degree of opacity, and/or a distribution of opacity. The method includes characterizing the optical inhomogeneity using the one or more volume segments.

In an embodiment, characterizing the optical inhomogeneity type comprises using a decision tree.

In an embodiment, characterizing the optical inhomogeneity type includes using a machine learning model.

In an embodiment, the machine learning model receives, as an input, the optical coherence tomography data and the image data and generates, as an output, the optical inhomogeneity type.

In an embodiment, the machine learning model comprises a convolutional neural network.

In an embodiment, the machine learning model is trained using supervised learning and using a training dataset comprising optical coherence tomography data, image data, and a labelled optical inhomogeneity type of a plurality of patients.

In an embodiment, the method further comprises displaying, on a display, an indicator of the optical inhomogeneity type. Optionally, the optical coherence tomography data and/or the image data is displayed.

In an embodiment, the method further comprises displaying, on the display, the one or more area segments overlaid on the optical coherence tomography data and/or the image data.

In an embodiment, the method further comprises generating one or more activation maps of the neural network. The method comprises displaying, on the display, the one or more activation maps. Preferably, the one or more activation maps are overlaid on the optical coherence tomography data and/or on the image data.

The activation maps can be displayed as color scale overlay. For example, using heat map colors. This enables a visual check of the characterized optical inhomogeneity in the eye and in particular improves the comprehensibility of the neural network output.

In an embodiment, the method further comprises adapting a treatment pattern for laser treatment of the eye using the optical inhomogeneity type, the optical coherence tomography data, and/or the image data.

In an embodiment, the treatment pattern comprises a sequence of treatment points, each treatment point having associated therewith a location and laser beam parameters. Adapting the treatment pattern comprises adjusting the location of one or more treatment points and/or adjusting the laser beam parameters for one or more treatment points. The adjustment of the location and/or laser beam parameters depends on the cataract grade, the optical coherence tomography data, and/or the image data.

The laser beam parameters include the laser beam power, pulse energy, pulse duration, spot diameter, spot spacing, repetition rate, and/or wavelength.

In an embodiment, the method further comprises receiving supplementary patient information including an age of the patient, a sex of the patient, a visual acuity of the eye of the patient, one or more symptoms of the patient, and/or a medical history of the patient. Characterizing the cataract grade further comprises using the supplementary patient information. The symptoms may include glare, halo, and/or photosensitivity.

In addition to a computer-implemented method for characterizing an optical inhomogeneity in a human eye, the present disclosure also relates to ophthalmological imaging system. The ophthalmological imaging system comprises a processor, an optical coherence tomography system, a camera, and a lighting system. The processor is configured to perform the method as described herein.

In addition to a computer-implemented method for characterizing an optical inhomogeneity in a human eye and an ophthalmological imaging system, the present disclosure also relates to a laser treatment device. The laser treatment device comprises a base station having a laser source configured to generate a laser beam, an arm connected to the base station configured to provide a beam path for the laser beam, an application head configured to direct the laser beam into an eye of a patient, and an ophthalmological imaging system as described herein.

The present disclosure also relates to a computer program product comprising computer program code configured to control a processor such that the processor performs the method as described herein.

The present disclosure also relates to a non-transitory computer-readable medium having stored thereon computer program code configured to control a processor such that the processor performs the method as described herein.

The present disclosure also relates to a training dataset including training OCT data and training image data from a plurality of eyes having an optical inhomogeneity, the training dataset further including a label identifying the optical inhomogeneity and optionally identifying features present in the training OCT data and the training image data. Preferably, the training dataset is augmented by generating additional OCT data using the training OCT data and adding known OCT data artifacts, and also by generating additional image data using the training image data and adding known image data artifacts.

### BRIEF DESCRIPTION OF THE DRAWINGS

The herein described disclosure will be more fully understood from the detailed description given herein below and the accompanying drawings, which should not be considered limiting to the invention described in the appended claims. The drawings in which:
- Fig. 1: shows a block diagram illustrating schematically an ophthalmological imaging system;
- Fig. 2: shows a block diagram illustrating schematically a laser treatment device comprising an ophthalmological imaging system;
- Fig. 3: shows an illustration of a patient in front of an ophthalmological imaging system;
- Fig. 4: shows an illustration of a patient reclined underneath an application head of a laser treatment device;
- Fig. 5: shows OCT data, in particular an OCT B-scan, of an eye having a cataract;
- Fig. 6: shows OCT data, in particular an OCT B-scan, of an eye having a cataract, with an activation map overlaid on the OCT B-scan indicating which areas of the OCT B-scan were identified by the machine learning model as contributing to the identification of a cataract;
- Fig. 7: shows OCT data, in particular an OCT B-scan, of an eye having a cataract, with outlines of areas overlaid on the OCT B-scan indicating the areas of the OCT B-scan identified to have a cataract;
- Fig. 8: shows a flow diagram illustrating an exemplary collection of input data of an eye including OCT data, first image data, and second image data, the input data being provided to an image processor configured to characterize the one or more types of optical inhomogeneity present in the eye;
- Fig. 9: shows a flow diagram illustrating an image processor configured to characterize the one or more types of optical inhomogeneity present in the eye, the image processor receiving OCT data and one or more types of image data and generating an output comprising the one or more types of optical inhomogeneity present in the eye;
- Fig. 10: shows a flow diagram illustrating a method for characterizing an optical inhomogeneity using OCT data and image data;
- Fig. 11: shows a flow diagram illustrating a method for characterizing an optical inhomogeneity using OCT data and image data, and displaying an indicator of an optical inhomogeneity type including optionally displaying a data overlay;
- Fig. 12: shows a flow diagram illustrating a method for characterizing an optical inhomogeneity using OCT data and image data, including the identification of area segments;
- Fig. 13: shows a flow diagram illustrating a method for characterizing an optical inhomogeneity using OCT data and image data, including the generation of a 3D representation of part of the eye and the identification of volume segments;
- Fig. 14: shows a flow diagram illustrating a method for adapting a treatment pattern using the optical inhomogeneity type; and
- Fig. 15: shows a diagram illustrating the training of a machine learning module used to characterize the optical inhomogeneity type.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

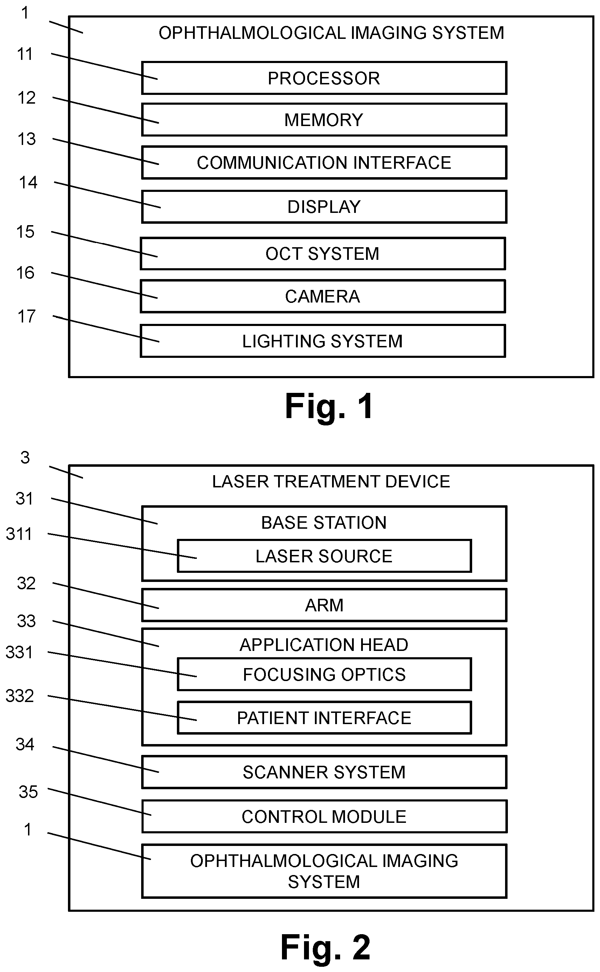

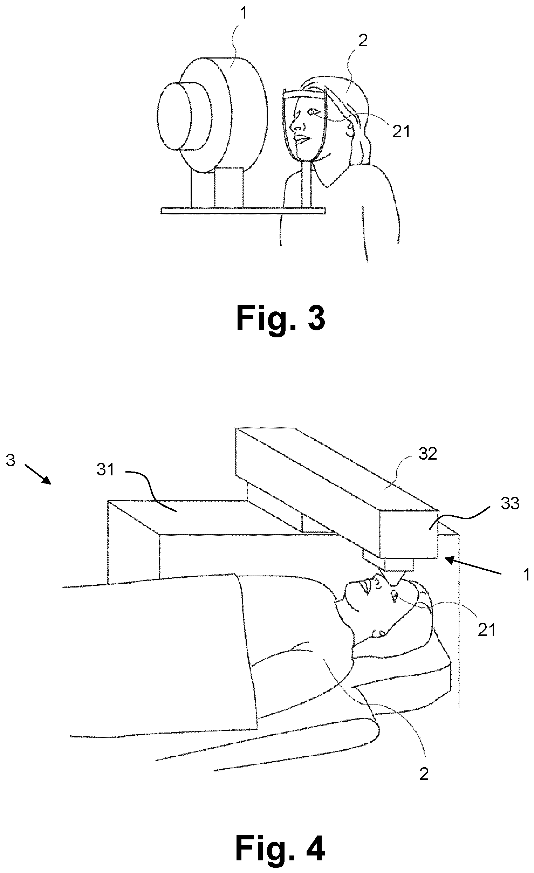

**Fig. 1** shows a block diagram illustrating schematically an ophthalmological imaging system 1. The ophthalmological imaging system 1 is used by an eye treatment professional, for example an optometrist or ophthalmologist for recording and optionally storing one or more images of the eye 21 of a patient 2. The ophthalmological imaging system 1 may be implemented as a desktop system which the patient sits in front of, as depicted in **Fig. 3** or as part of a laser treatment device as depicted in Fig. 4
The ophthalmological imaging system 1 comprises a processor 11, a memory 12, a communication interface 13, and a user interface, which are communicatively coupled to each other either directly or indirectly.

The processor 11 comprises one or more processors, e.g. implemented as one or more central processing units (CPUs). The processor 11 may include other programmable circuits or logic units such as ASICs (Application-Specific Integrated Circuits), GPUs (graphics processing units) and TPUs (tensor processing units).

The memory 12 comprises volatile and/or non-volatile memory modules, e.g., random-access memory and/or flash memory. The memory 12 has stored thereon program code (compiled and/or uncompiled program code, for example in the form of one or more software applications (Apps)), data, software libraries, as well as programmed software modules for controlling the processor 11. The processor 11 performs the methods (in particular, one or more steps and/or functions) as described herein according to instructions of the program code and/or due to the processor 11 being specifically programmed to carry out such instructions. As such, the processor 11 is configured to perform the methods, steps, and/or functions as described herein.

The memory 12 is further configured to receive and/or store OCT data and image data of an eye of a patient 2. The memory 12 may further include control data relating to a treatment pattern used to treat an eye 21 of a patient 2.

In an embodiment, the memory 12 is further configured to store a trained machine learning model, for example comprising a decision tree and/or a neural network, for use in one of the methods described herein.

The communication interface is further configured for data communication with one or more external devices. Preferably, the communication interface comprises a network communications interface, for example an Ethernet interface, a WLAN interface, and/or a wireless radio network interface for wireless and/or wired data communication using one or more networks, comprising, for example, a local network such as a LAN (local area network), and/or the Internet.

The skilled person is aware that at least some of the steps and/or functions described herein as being performed on the processor 11 of the ophthalmological imaging system 1 may be performed on one or more auxiliary processing devices connected to the processor 11 of the ophthalmological imaging system 1 using the communication interface. The auxiliary processing devices can be co-located with the ophthalmological imaging system 1 or located remotely, for example on a remote server computer.

The skilled person is also aware that least some of the data associated with the program code (application data) or data associated with a particular patient (patient data) and described as being stored in the memory 12 of the ophthalmological imaging system 1 may be stored on one or more auxiliary storage devices connected to the ophthalmological imaging system 1 using the communication interface.

The user interface comprises, for example, one or more user input devices, such as a keyboard, mouse, trackpad, mouse, pen, touch-input etc. The user interface comprises one or more output devices, such as a display 14 (e.g. an LCD screen or an OLED screen), one or more loudspeakers, or an extended reality (e.g., augmented reality or virtual reality) system. The user interface is configured to receive user inputs from an eye treatment professional, in particular based on, or in response to, information displayed to the eye treatment professional using the one or more output devices.

The ophthalmological imaging system 1 further includes a measuring system comprising an interferometric measuring device, in particular an optical coherence tomography (OCT) system 15 configured to record OCT data of the eye of a patient. Specifically, the OCT system 15 is configured to record one or more A-scans, B-scans, and/or C-scans of the eye of a patient. The B-scans of the eye are 2D images of the eye typically having one axis parallel to an optical axis of the OCT system and one axis perpendicular to the optical axis. The B-scans may alternatively also be an accumulation of A-scans along a continuous path lateral to the optical axis. The B-scans may be arranged parallel to each other, such that a series of B-scans provide information about the whole (or part of) the 3D volume of the eye. The B-scans may also be arranged rotated with respect to one another other, i.e. rotated about the optical axis, such that a series of B-scans, when viewed along the optical axis, are arranged like spokes on a wheel and thereby also provide information about the whole (or part of) the 3D volume of the eye. For example, 100 B-scan "slices" may be recorded in a 180° sweep about the optical axis, thereby providing OCT data of the total 360°. Additionally, or alternatively, information about the 3D volume of the eye may be provided by a point cloud including a large number of A-scans (e.g., several thousands). Additionally or alternatively, A-scans arranged in a spiral may also provide information about the 3D volume of the eye. Alternatively to A-scans, T-scans may also be recorded and used as a basis for the B-scans and C-scan.

The data acquired by the OCT system 15 relates in particular to the following parts and structures of the eye: the cornea, iris, ciliary body, posterior pigment epithelium, anterior capsule, lens, and/or posterior capsule.

The OCT system 15 is configured to perform, for example, time domain optical coherence tomography (TD-OCT) (e.g., time-domain anterior segment OCT (AS-OCT) (at 1300 nm)), spectral domain optical coherence tomography (SD-OCT) (e.g., spectral-domain anterior segment optical coherence tomography (AS-OCT) system (at 820 nm), ultrahigh speed swept source optical coherence tomography, ultrahigh resolution optical coherence tomography, polarization sensitive optical coherence tomography, and/or adaptive optics optical coherence tomography data.

The number (or equally, the density) of B-scans may lie in the range of up to thousands, depending on the particular configuration of the OCT system 15.

In an embodiment, a small number of 1 - 10 OCT B-scans are acquired at several discrete angles around the optical axis.

In an embodiment, a high number of 100 - 1000 OCT B-scan 'slices' are combined into a volumetric scan pattern (e.g., in the form of a three dimensional C-Scan) of the eye.

In an embodiment, 10'000 - 100'000 A-scans may be combined to generate the volumetric scan pattern.

The resolution of the OCT system 15 is preferably below 50 micrometers in a lateral direction.

The ophthalmological imaging system 1 further includes a camera 16 and a lighting system 17. The camera 16 (e.g. comprising a photodetector array, such as a CMOS or CCD array), is configured to record one or more color and/or hyperspectral images of the eye, including in particular infrared images up to 1350 nm. The camera 16 is also understood to include one or more optical systems configured for recording a directly illuminated image of the eye, a retroilluminated image of the eye, and/or a Scheimpflug image of the eye.

The lighting system 17 is connected to the camera 16 and configured for one or more imaging techniques. The imaging techniques include direct illumination of the eye, retroillumination of the eye, and/or Scheimpflug imaging.

The camera 16 in conjunction with the lighting system 17 is configured to record image data of the eye including one or more directly illuminated images, one or more retroilluminated images, and/or one or more Scheimfplug images.

**Fig. 2** shows a block diagram illustrating schematically a laser treatment device 3 for treating eye tissue by means of laser pulses, for example the cornea or another tissue of an eye 21 of a person 2. The laser treatment device 3 is also illustrated in **Fig. 4****.** The laser treatment device 3 comprises an ophthalmological imaging system 1. The laser treatment device 3 comprises a base station 31 having a laser source 311, an arm 32 for guiding a treatment laser beam from the laser source 311 along a beam path, an application head 33 having focusing optics 331 and a patient interface 332.

The laser source 311 is configured to generate a pulsed laser beam L. The laser source 311 comprises in particular a femtosecond laser for generating femtosecond laser pulses, which typically have pulse widths ranging from 10 fs to 1000 fs (1 fs = 10⁻¹⁵ s).

The scanner system 34 is configured to steer the pulsed laser beam delivered by the laser source 311 by means of the focussing optics 331 in the eye tissue onto treatment points on a treatment pattern (comprising a laser trajectory). In an embodiment, the scanner system 34 comprises a divergence modulator for modulating the focal depth, or the treatment height, in the projection direction along the projection axis p. The scanner system 34 comprises, for example, a galvanoscanner or a piezo-driven scanner. Depending on the embodiment, the scanner 34 additionally comprises one or more deflecting mirrors, one or more resonant mirrors, or one or more oscillating mirrors, which are for example piezo-driven, or MEM (Micro-Electromechanical), or the scanner system 34 comprises an AOM (Acousto-Optical Modulator) scanner or an EOM (Electro-Optical Modulator) scanner.

The application head 33 comprises focussing optics 331 and a patient interface 332. The application head 33 can be placed onto the eye 21 and fixed on the eye 21 by means of the patient interface 332.

The focussing optics 331 are configured to focus the pulsed laser beam L, or its laser pulses, onto the treatment points inside the eye tissue for the pointwise tissue disruption. The focussing optics 331 comprise a lens system having one or more optical lenses. Depending on the embodiment, the focussing optics 331 comprise one or more movable lenses and/or a drive for moving the entire focussing optics 331 in order to set and adjust the focal depth, or the treatment height, in the projection direction along the projection axis p. In a further embodiment, a divergence modulator is provided in the beam path between the laser source 311 and the scanner system 34.

The treatment laser device 3 is configured for so-called laser phaco treatment. In particular, the treatment pattern is configured such that a cataract is broken into several pieces along with an incision made such that the cataract may be suctioned out and removed. After the cataract is removed, an intraocular lens is typically inserted in order to replace the natural lens. The cut eye tissue at the site of the original incision heals itself without the need for any sutures. Ultrasonic phaco treatment may be used in addition to emulsify the pieces of the cataract.

The laser treatment device 3 comprises a control module 35 for controlling the laser treatment device 3. The control module 35 is configured to control the laser source 311, the optical functional module of the scanner system 34, the focusing optics 331, and the ophthalmological imaging system 1 described above. The control module 35 embodies a programmable control device and comprises, for example, one or more processors (for example as described herein with reference to the processor 11 of the ophthalmological imaging device 1) and a memory (for example as described herein with reference to the memory 12 of the ophthalmological imaging device 1) The control module is 35 connected to the various components of the laser treatment device using a communication interface. The memory may be configured to store control data relating to a treatment pattern used to treat an eye 21 of a person 2.

The ophthalmological imaging system 1 is functionally and optionally integrated in the laser treatment device 1. For example, the measuring device including the OCT system, camera 16 and lighting system 17 is integrated, at least in part, into the application head 33, such that the patient 2 reclining underneath the application head may have OCT data and/or image data of an eye 21 recorded. The processor 11, memory 12, and communication interface 13 may be integrated into the base station 31 of the laser treatment system 3.

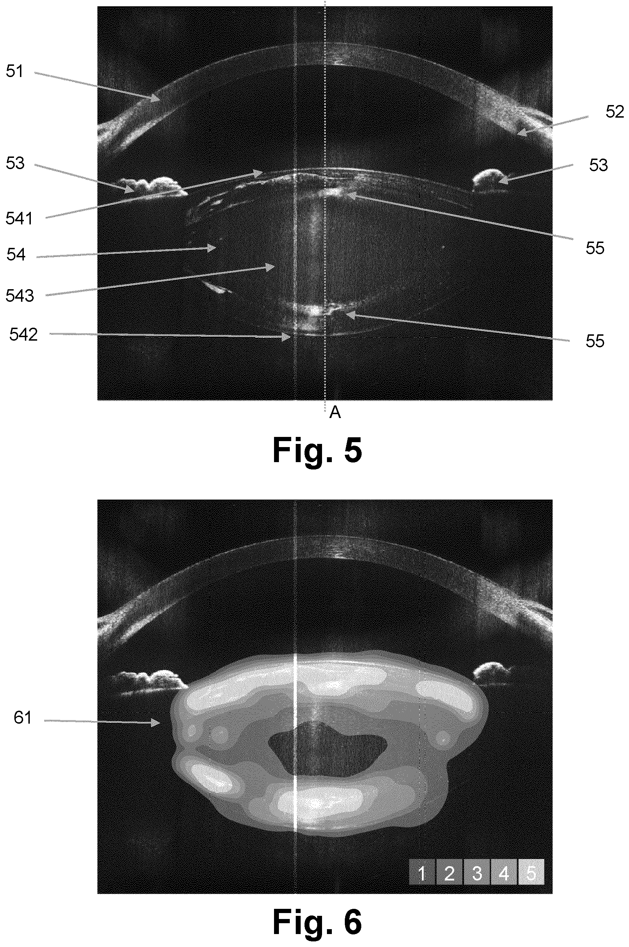

Fig. 5 shows a graphical representation of OCT data, in particular an OCT B-scan, of an eye having a cataract. The OCT B-scan shows the cornea 51, sclera spur 52, iris 53 and lens 54 of the eye. The lens 54 includes a lens anterior capsule 541, a lens posterior capsule 542, and a lens substance 543. Visible in the B-scan are also areas of opacity 55 due to the cataract. The OCT data typically includes a series of such B-scans to provide information about the whole eye. The B-scans may be rotated with respect to each other about the optical axis A, or shifted with respect to each other laterally such that the third dimension is captured in a series of B-scan "slices".

Fig. 6 shows a representation of OCT data as depicted in Fig. 5, with an activation map 61 overlaid on the OCT B-scan indicating which areas of the OCT B-scan were identified by the machine learning model described herein as contributing to the identification of the cataract. The activation map 61 as depicted includes five gradations indicating with more granularity which parts of the OCT B-scan the machine learning model responded to the most.

Such a representation may be displayed for the eye treatment professional, in particular an ophthalmologist in particular on the display 14, such that the eye treatment professional may comprehend what lead to the particular characterization and readily judge the quality or trustworthiness of the characterization.

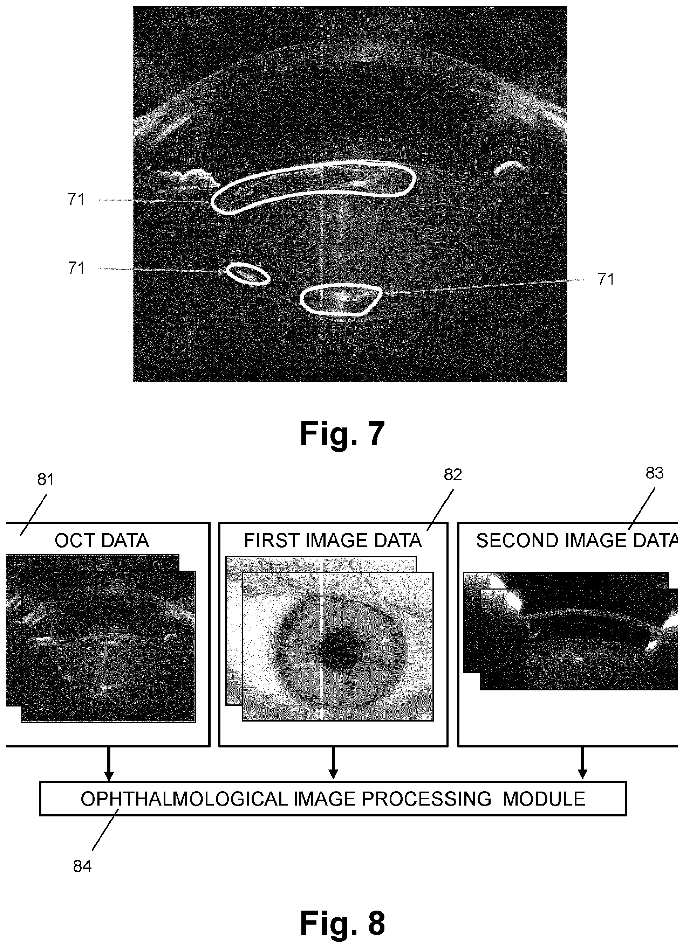

**Fig. 7** shows a representation of OCT data as depicted in **Fig. 5****,** with overlaid outlines of area segments 71 corresponding to areas of opacity in the eye, as determined by one or more of the methods described herein.

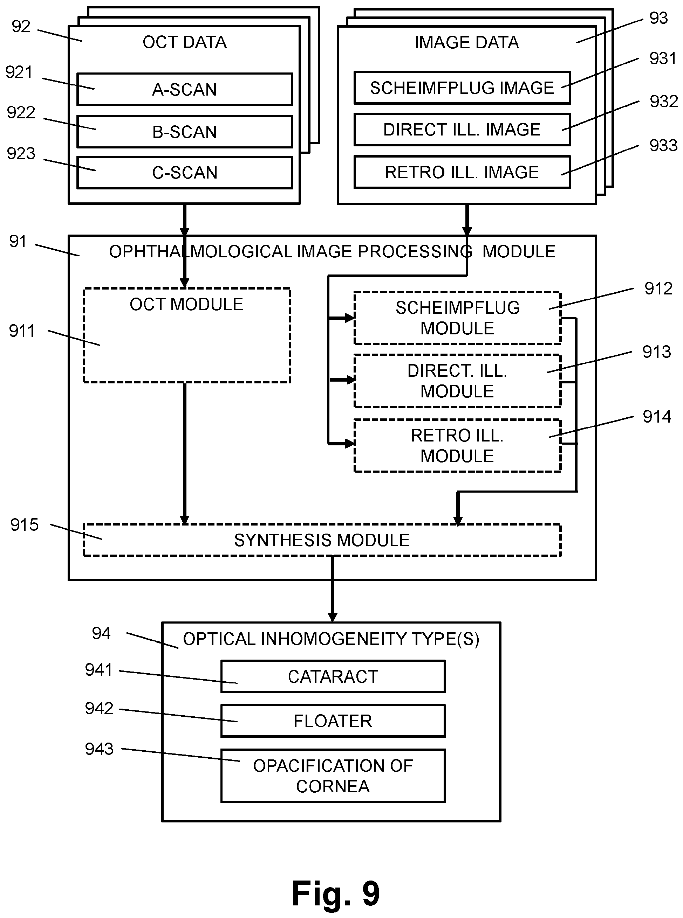

**Fig. 8** shows a flow diagram illustrating an exemplary collection of input data including OCT data 81, first image data 82, and second image data 83. The input data is input into an image processor 81 configured to generate an output. The output includes a characterization of one or more types of optical inhomogeneity in the eye. The image processor 84, 91 is described in more detail with reference to **Fig. 9****.**

**Fig. 9** shows an exemplary embodiment of an ophthalmological image processing module 91. The ophthalmological image processing module 91 is implemented as one or more software applications and/or software libraries and includes algorithms and/or models for image processing.

The ophthalmological image processing module 91 is used to process two dimensional and/or three dimensional data, in particular OCT data 92 and image data 93.

The ophthalmological image processing module 91 may be stored in the memory 12 and executed by the processor 11. The ophthalmological image processing module 91, or parts thereof, may alternatively be stored and/or executed in a remote server computer. In particular, modules 911, 912, 913, 914 of the ophthalmological image processing module 91 may comprise machine learning models, or other data structures or algorithms used to process the OCT data 92 and/or the image data 93. These machine learning modules, libraries, models, and/or algorithms, are stored locally with respect to the processor 11, i.e. the memory 12, or other device performing the method. The algorithms, machine learning models, or other data structures may alternatively also be stored remotely and retrieved by the processor 11 on demand. Additionally or alternatively, the processor 11 may transmit particular data to a remote machine hosting the machine learning modules, libraries, models, and/or algorithms and receive processed data from the remote machine.

The ophthalmological image processing module 91 is typically configured to receive, as an input, OCT data 92 and/or image data 93 of a particular eye and generate, as an output, one or more optical inhomogeneity types 94 present in the particular eye. The OCT data 92 includes a plurality of A-scans 921, B-scans 922, and/or C-scans 923 of the eye. The image data 93 comprises a plurality of Scheimpflug images 932, directly illuminated images 932, and/or retro illuminated images 933. The optical inhomogeneity types include, for example, cataracts 941, floaters 942, and/or an opacification of the cornea 943.

The ophthalmological image processing module 91 may include an OCT module 911 specific to OCT data, and one or more image modules 912, 913, 914 specific to the image data, in particular specific to each type of image data (i.e. whether the image data relates to an image of a Scheimpflug illuminated eye, directly illuminated eye, or retro illuminated eye). Each module 911, 912, 913, 914 may include one or more models and/or algorithms.

Specifically, the OCT module 911 is configured to receive, as an input, the OCT data 92. The Scheimpflug module 912 is configured to receive, as an input, the Scheimpflug image(s) 931. The directly illuminated module 913 is configured to receive, as an input, the retro-illuminated image(s) 932. The retro-illuminated model 914 is configured to receive, as an input, the retro-illuminated image(s) 933.

The output from the modules 911, 912, 913, 914 is passed to a synthesis module 915 which is configured to combine the outputs from each of the modules 911, 912, 913, 914 and characterize the one or more types of optical inhomogeneity present in the particular eye.

The modules 911, 912, 913, 914 may also alternatively be combined into a single module configured to process the OCT data 92 and the image data 93 in parallel, for example implemented as a neural network.

The modules 911, 912, 913, 914, as described in more detail below, are configured to identify features in the OCT data 92 and/or the image data 93. The features may include global features and/or local features. The features may include areas and/or volumes of opacity, which may be linked to one or more global features and/or local features. The features are indicative of one or more types of optical inhomogeneity.

The features may include normal anatomical features of the eye, in particular those features discussed herein with reference to **Fig. 5****.** Abnormal features, i.e. those which may be indicative of an optical inhomogeneity, may be identified with reference to a particular anatomical feature, i.e. the relative location of an abnormal feature may be identified with respect to an anatomical feature, for example as occurring in, on, or adjacent to a particular anatomical feature.

Global features are related to properties of the eye indicative of an optical inhomogeneity which may not have a precisely defined position, such as a general haziness, cloudiness, or inflammation, in particular of the cornea, the lens anterior capsule 541, the lens posterior capsule 542, and/or the lens substance 543 as shown in **Fig. 5****.** These global features may be identified using a tonal distribution of the OCT data 92 and/or image data 93 (or parts thereof), in particular using a non-local dynamic range, a non-local contrast level, etc.

Local features are related to properties of the eye indicative of an optical inhomogeneity which have a defined location. Local features include in particular floaters, corneal scars, or posterior subcapsular cataracts. These features may be identified as spots and/or areas of brightness and/or differing or particular color, contrast, structuring, or texture indicative of an optical inhomogeneity.

Floaters are not scattering by nature but refract light rays differently so that images on the retina are locally black because the light is refracted in other directions. Scattering tissue tends to be more large-volume and fogs the entire image that forms on the retina. Therefore, contrast is reduced globally.Depending on the embodiment, the ophthalmological image processing module 91 and/or the image processing modules 911, 912, 913, 914 are configured to identify area segments of opacity in the OCT data 92 and/or in the image data 93.

Optionally, the area segments are defined by, or include, properties which include geometric parameters such as a location of an area segment, a width and a height of an area segment, a shape of an area segment, and/or size of an area segment, etc. The area segments may further be defined by, or include as one of the geometric properties, one or more regions in the image, e.g. defined by one or more bounding curves, bounding boxes, and/or outlines. The geometric properties may further include properties related to the spatial arrangement of two or more identified area segments in the OCT data and/or in the image data, in particular a distance between a particular image segment and another image segment, and a relative location between a particular image segment and another image segment.

The properties may further include optical parameters such as a degree of opacity in one or more parts of the area segment, a distribution of opacity, a variation of opacity, a measure of contrast, a measure of structuring, a brightness, and a measure of structure compared to the neighborhood of the area segment.

In an embodiment, the ophthalmological image processing module 91 and/or the image processing modules 911, 912, 913, 914 are configured to identify volume segments of opacity in the OCT data 92 and/or in the image data 93. The ophthalmological image processing module 91 may be combined, for example, to link an area segment identified in a particular B-scan 922 of the OCT data 92 with an area segment identified in an adjacent B-scan 922, thereby generating a volume segment. The same applies to joining area segments identified in the image data 93. The ophthalmological image processing module 91 may, alternatively or additionally, be configured to generate a three dimensional representation of the eye using the OCT data 92 (often referred to as a C-scan) and then to identify the volume segments of opacity within the three dimensional representation. The three dimensional representation may be generated using a plurality of one dimensional A-scans and/or two dimensional B-scans. The same applies to a three dimensional representation of the eye generated using the image data 93 (in particular, the Scheimpflug images 931).

In particular, the volume segments are regions of space which have a substantial extension in all three dimensions, such that the volume segments do not correspond to two-dimensional slices of mere nominal thickness. In an embodiment, a given tissue layer or fluid layer in the lens area of the eye is represented in a single volume segment.

The volume segments may be defined by, or be linked to, features, such as local features. Additionally, the volume segments may have properties such as geometric properties. The geometric properties comprise a height of a given volume segment, a width of the given volume segment, a spatial arrangement of the given volume segment with respect to other volume segments, a volume of the given volume segment, a thickness of the given volume segment, a shape of the volume segment, and a measure of how smooth the surface of the given volume segmentis.

The properties may further include optical parameters such as a degree of opacity in one or more parts of the volume segment, a distribution of opacity, a variation of opacity, a measure of contrast, a brightness, a measure of structuring (for example relative to a neighborhood of the volume segment), etc. These geometric properties are generated by the ophthalmological image processing module 91.

The properties of the area segments and/or volume segments may further include material properties of the segments deduced from the OCT data 92 and/or the image data 93, in particular a level of hardness, compaction, toughness, and/or density, and optionally a spatial distribution or variance of such material properties in the segments.

The synthesis module 915 is configured to combine the outputs of the dedicated modules 911, 912, 913, 914, in particular to perform a kind of "sensor fusion" to establish a more complete, accurate, and/or robust representation of the eye and, in particular, to enable the better characterization one or more types of optical inhomogeneity which may be present in the eye. For example, OCT-data 92 in particular readily indicates edges and boundaries of tissues or abnormalities within them due to the associated transitions in the refractive index. Directly illuminated (front on view) colour images are suitable for determining changes in colour in the eye. Scheimpflug images, on the other hand, respond best to differences in local intensity of optical scattering.

The details of the synthesis module 915 depend the specifics of the outputs of the modules 911, 912, 913, 914. For example, the synthesis module 915 may compare the list of features identified in the OCT data 92 with the features identified in the image data 93.

The synthesis module 915 may establish a complete list of features (global and/or local) using the features identified in the OCT data 92 and the image data 93. Thereby, features identified in the OCT data 92 but not apparent in the image data 93 (or vice versa) are not missed. For example, a complete list of area and/or volume segments is established by the synthesis module 915 using the area and/or volume segments identified in the OCT data 92 and the image data 93. The synthesis module 915 is configured to use the complete lists of features, area segments and/or volume segments to characterize the optical inhomogeneity.

Additionally or alternatively, the synthesis module 915 is configured to identify and exclude features identified in the OCT data 92 and not identified in the image data 93 or vice versa, thereby avoiding false positive detections of inhomogeneities. If a plurality of types of image data 93 are received, the synthesis module 915 may implement a voting mechanism to include features for use in characterization on if they are, for example, present in a majority, supermajority, or all of data sources (i.e. in the OCT data 92 and the plurality of types of image data 93).

The synthesis module 915 may be configured to weight the robustness of one or more features detected in the source data (i.e. the OCT data 92 and/or in the image data 93, in particular for each type of image data 931, 932, 933). The robustness is weighted using a weight table which associates, for a particular type of source data (i.e. for the OCT data 92 or for the image data 93), a particular feature with a weight indicative of the robustness of which the particular feature is in principle identifiable in the particular source data. Thereby, features detected in the OCT data 92 for which OCT data is in principle less reliable are weighted less than the same feature detected in the image data 93, for example. The weighting procedure may, for example, include knowledge about the particular measuring device and their associated measuring reliability or strengths and weaknesses. As an example, the OCT data 92 result may be ranked higher for local inhomogeneities due to abrupt changes in refractive index, compared to image data 931 coming from a Scheimpflug camera system which is more reliable for detecting diffuse inhomeneities such as haze. This lowers the rate of false positive feature identification and increases the robustness of the characterization.

Additionally or alternatively, the outputs of the modules 911, 912, 913, 914 may already comprise a characterization of the optical inhomogeneity. The synthesis module 915 may then be configured to identify one or more commonalities between the outputs of the modules 911, 912, 913, 914 to provide a more accurate and robust characterization of the optical inhomogeneity present in the eye.

The ophthalmological image processing module 91, in particular the modules 911, 912, 913, 914, 915 may be implemented as described below.

The ophthalmological image processing module 91, in particular the modules 911, 912, 913, 914, 915, may comprise a decision tree. The decision tree may be configured to segment and/or classify the OCT data 92 and/or the image data 93 either in whole or in part. The synthesis module 915 may also comprise a decision tree. The decision tree is either specifically programmed or trained using machine learning. The (programmed or trained) decision tree may be used to identify features in the OCT data 92 and/or the image data 93, and/or to identify area and/or volume segments and properties thereof.

The ophthalmological image processing module 91, in particular one or more of the modules 911, 912, 913, 914, 915, may comprise a random forest classifier. The random forest classifier may be configured to segment and/or classify the OCT data 92 and/or the image data 93 either in whole or in part. The (programmed or trained) random forest classifier may be used to identify features in the OCT data 92 and/or the image data 93, and/or to identify area and/or volume segments and properties thereof.

The ophthalmological image processing module 91, for example comprising one or more of the modules 911, 912, 913, 914, 915, may comprise a neural network, in particular a convolutional neural network. The neural network preferably comprises a U-Net and/or a ResNet. In particular, ResNet18, ResNet50 have shown good results. Likewise, similar neural networks including EfficientNet-B0, EfficientNet-B4 and EfficientNet-B6 have also shown good results. EfficientNet incorporates so-called compound scaling, a combination of depth (number of layers), width (width of layers), and resolution (resolution of input images) scaling. These networks perform well compared to state-of-the-art models with respect to accuracy while using fewer parameters and FLOPS. The U-Net is a convolutional neural network developed for biomedical image segmentation with an architecture designed to yield precise and accurate results with fewer training images than classical fully-connected convolutional neural networks. It achieves this by having, in addition to a contracting part, which detects small features, an up-sampling part which increases the resolution of the output image and which receives contextual information from many of the contracting layers, generating a high resolution output. ResNet is a neural network architecture which uses shortcut connections to selectively skip one or more layers, allowing efficient training of deep convolutional neural networks.

The synthesis module 915 may be implemented using one or more of the models described above, particularly a decision tree, a random forest and/or a neural network configured as a classifier. The synthesis module 915 may be further configured to receive the supplementary patient information as an additional input and to characterize the optical inhomogeneity further using the patient information.

Further specific properties and functions of the ophthalmological image processing module 91, and other optional arrangements and modules of the ophthalmological image processing module 91, are discussed below with reference to methods which use the ophthalmological image processing module 91.

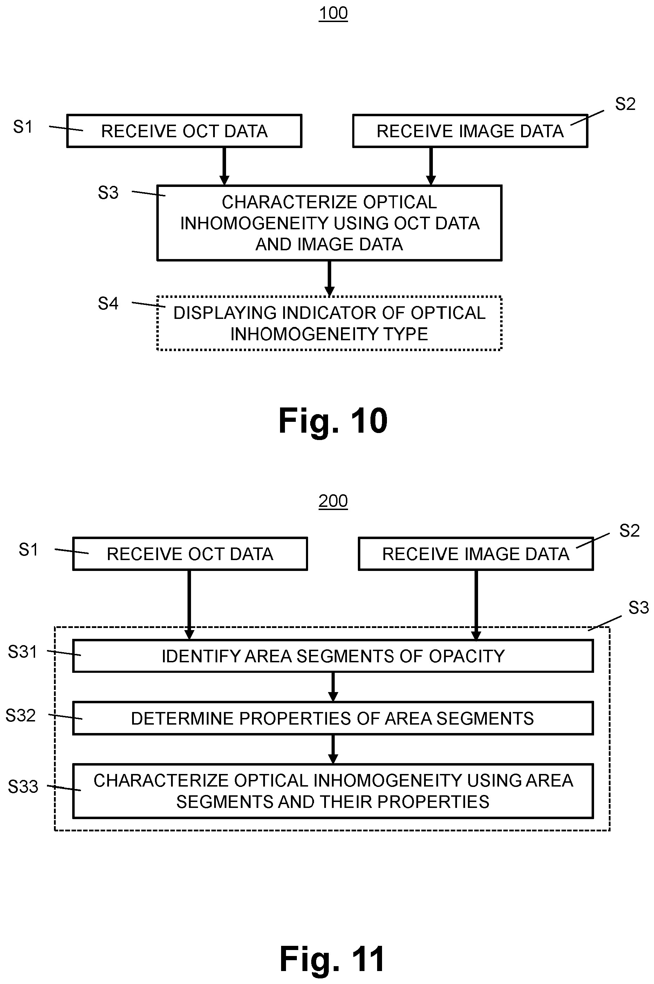

**Figs. 10** to **14** show flow diagrams illustrating methods 100 - 500 for characterizing an optical inhomogeneity in the eye 21 of a patient 2. The methods 100 - 500 include a number of steps which are not necessarily required to be performed in the sequence illustrated. The methods 100 - 500 may be performed by the processor 11 of the ophthalmological imaging device 1. The methods 100 - 500 may also be performed by a computing device including, for example, a remote server computer (belonging to, for instance, a cloud computing center). The methods 100 - 500 may be performed on demand, initiated by a user (i.e. an eye treatment professional), or may be initiated by a prompt, trigger, or other signal from a computer process executing on the same machine or on a connected machine.

The methods 100 - 500 use the ophthalmological image processing module 91 as described with reference to **Fig. 9****.**

**Fig. 10** shows a flow diagram of the method 100 including steps S1 - S4.

In a preparatory step, the ophthalmological imaging device 1 records OCT data of the eye 21 using the OCT system 15. The OCT data comprises one or more A-scans and/or one or more B-scans. The ophthalmological imaging device 1 further records image data of the eye 21 using the camera 16 and the lighting system 17. The image data may correspond to one or more images of the eye 21 which are directly illuminated, one or more images of the eye 21 which are retro-illuminated, and/or one or more images of the eye 21 which are taken by Scheimpflug imaging.

In step S1, the OCT data is received. In step S2, the image data is received.

In addition to the OCT data and the image data, supplementary patient information may also be received from a database stored either locally or remotely. The supplementary patient information includes an age of the patient, a sex of the patient, a visual acuity of the eye of the patient, one or more symptoms of the patient, or a medical history of the patient.

In step S3, the OCT data and the image data is used to characterize an optical inhomogeneity in the eye. The characterization step includes a classification of the optical inhomogeneity and optionally a grading of the optical inhomogeneity. The characterization may also include a determination of whether an optical inhomogeneity is present in the eye at all. The optical inhomogeneity is characterized as being of one or more optical inhomogeneity types.

The optical inhomogeneity types include a cataract, a floater, and/or an opacification of the cornea. The cataract may also be characterized as being one of a plurality of types of cataracts. The cataract may be characterized as having a particular grade and/or severity.

The OCT data and the image data may be characterized using the ophthalmological image processing module 91.

In an embodiment, supplementary patient information is also used to characterize the optical inhomogeneity. The supplementary patient information may also be provided to the image processing module 91 which processes this information and uses it to characterize the optical inhomogeneity. As an example, as a mostly age-related disease, cataract severity is highly correlated to the patient age. Several population-based studies also show a higher prevalence of cataract-induced lens opacities in female patients.

In an embodiment, if the characterized optical inhomogeneity includes a cataract, then characterizing the optical inhomogeneity type includes characterizing a cataract grade of the cataract. The cataract grade includes a cataract type comprising a nuclear sclerotic cataract, a cortical spoking cataract, and/or a posterior subcapsular cataract. Optionally, the cataract grade includes a cataract severity level associated with at least one of the one or more cataract types.

In optional step S4, an indicator of the optical inhomogeneity type is displayed, for example on the display 14. The indicator can include one or more of: a text describing the optical inhomogeneity type, a graphical illustration indicative of the optical inhomogeneity type, or a symbol of the optical inhomogeneity type. The indicator is used by the eye treatment professional, in particular an ophthalmologist, purely for informational purposes and at most as a suggestion for an underlying clinical picture. Specifically, the eye treatment professional establishes any diagnosis using, as a reference, the indicator of the optical inhomogeneity type. Optionally, the OCT data (in particular in the form of one or more B-scans) and/or the image data is displayed on the display 14. For example, the OCT data and the image data are displayed side-by-side in addition to the indicator of the optical inhomogeneity type. Thereby, the eye treatment professional can easily compare the OCT data and the image data and, with additional reference to the indicator of the optical inhomogeneity type, form an opinion as to the underlying clinical picture and establish a diagnosis.

The indicator of the optical inhomogeneity type is optionally stored in the memory 12, in particular in association with a patient file, the patient file including, in particular, at least a part of the OCT data and/or a part of the image data.

**Fig. 11** shows a flow diagram of the method 200 including steps S1 - S3. Steps S1 - S2 are described above with reference to method 100. An example of step S3 as mentioned with reference to method 100 is described in the following in more. In particular step S3 includes sub steps S31 - S33. This method 200 may be performed, at least in part, using the ophthalmological image processing module 91.

In step S31, one or more area segments of opacity are identified. The area segments of opacity are regions in the OCT data and/or the image data which are indicative of an optical inhomogeneity, see the description of **Fig. 9** for further details related to the area segments.

Each area segment identified in the OCT data and/or the image data may be associated with a particular part of the OCT data (e.g., one or more A-scans and/or one or more B-scans) or a particular image of the image data. Thereby, during rendering and display of the area segments on the display 14, the eye treatment professional can comprehend more easily which area segments are associated with which OCT data and/or image data.

In step S32, properties of the area segments are determined. These properties include features of the area segments, in particular geometric features.

The output of the ophthalmological image processing module 91, in particular the modules, is then used to characterize the optical inhomogeneity in step S33.

Subsequent to step S33, the method 200 may include further steps such as step S4 described with reference to method 100.

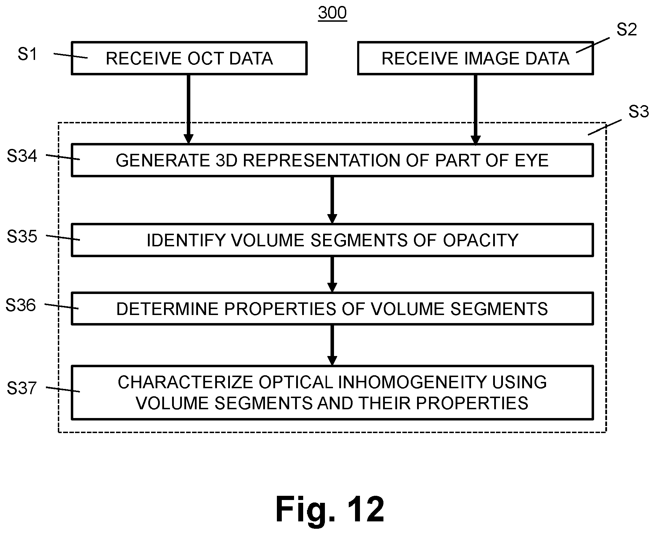

**Fig. 12** shows a flow diagram of the method 300 for characterizing an optical inhomogeneity using volume segments and optionally their properties. The method 300 includes steps S1-S3 previously described with reference to **Figs. 10** and **11****.** The method 300 further includes sub steps S34-S37 of S3. The method 300 may be performed using the ophthalmological image processing module 91 described with reference to **Fig. 9****.**

In an embodiment, the ophthalmological image processing module 91 comprises a neural network which is adapted to efficiently handle three-dimensional input data. Preferably, the neutral network comprises a U-Net and/or a ResNet architecture, as these two types of neural networks can be used and/or adapted to achieve good results in generating volume segments in three-dimensional data. The neural network may be used for one or more of the steps described below, in particular steps S35, S36, and S37.

In step S34, a three dimensional representation of part of the eye is generated. As described herein, the three dimensional representation may be generated using the OCT, in particular a sequence of OCT B-scans (which may be generated from OCT A-scans). The three dimensional representation may be a voxel model, each voxel having assigned thereto a particular set of values indicative, for example, of the brightness of a pixel in a corresponding B-scan. Other representations, including, for example, vector-based representations, volumetric meshes, and/or surface meshes, may also be used. Additionally, image data may be used to augment the three dimensional model, in particular image data from Scheimpflug imaging. Thereby, each voxel, for example, is associated OCT data and image data, achieving a more complete representation of the eye.

Alternatively, or additionally, a second three dimensional representation is generated using the image data, in particular image data from Scheimpflug imaging.

In step S35, features (global and/or local features) of the three dimensional representation(s) are identified. In particular, volume segments of opacity are identified within the three dimensional representation(s). The volume segments are preferably determined using the ophthalmological image processing module 91 which may implement a three dimensional segmentation algorithm. The volume segments may be defined by a set of adjacent voxels, surface meshes, or volume meshes.

In step S36, properties of the volume segments are determined. These properties are described in more detail with reference to **Fig. 9****.**

In step S37, the one or more types of optical inhomogeneity present in the eye are characterized using the volume segments and/or their properties.

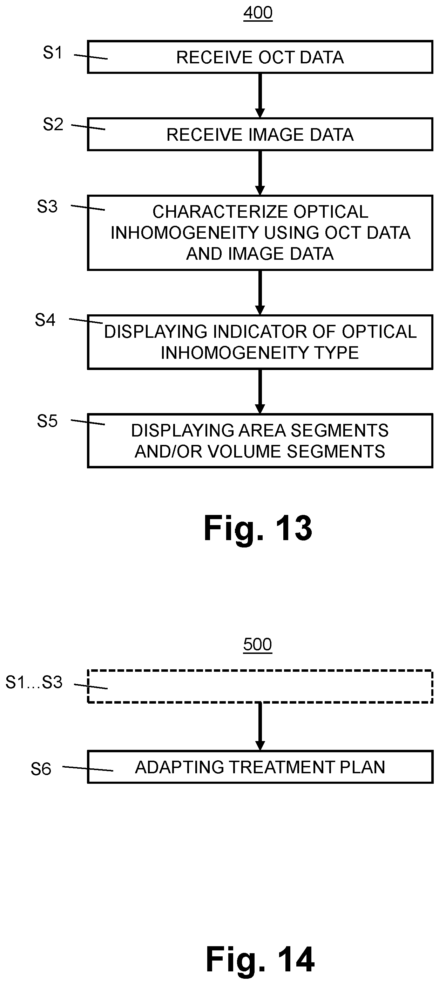

**Fig. 13** shows a flow diagram illustrating a method 400 for characterizing types of optical inhomogeneity in the eye. The method 400 includes steps S1-S5. Steps S1-S4 have previously been described with reference to **Fig. 10****.** The method may be implemented, at least in part, using the ophthalmological image processing module 91.

In step S5, the area segments and/or volume segments as identified in step S31 (as shown in Fig. 11) and in step S35 (as shown in Fig. 12) are rendered and displayed on the display 14. In particular, the area segments and/or volume segments are displayed in conjunction with the relevant OCT data and the relevant image data. Preferably, the indicator of the type of characterized optical inhomogeneity is also displayed on the display 14.

For example, the area segments are displayed overlaid on the relevant OCT B-scan, for example with a particular transparency such that the underlying OCT B-scan is still remains at least partially visible. The rendering and display of the area segments over the OCT B-scan allows the eye treatment professional to judge the accuracy and reliability of the indicated optical inhomogeneity type and establish a clinical picture according to his or her expertise along with the information provided by the indicator.

Depending on the output from the ophthalmological image processing module 91 and/or other modules related to characterizing the optical inhomogeneity type, the area segments may be rendered using a heat map or other graded map. In particular, the heat map can be configured to indicate which area segments were particularly relevant for the characterization. Thereby, the eye treatment professional can more easily comprehend the output and confirm whether the type of ophthalmological inhomogeneity was accurately characterized.

Additionally or alternatively, the area segments may be displayed by means of rendering an outline of each area segment, a bounding box surrounding a particular area segment, and/or or a graphical indicator element indicating a location of the area segment.

In an example, in which the ophthalmological image processing module 91 includes a neural network, the area segments may be rendered with an activation map overlaid indicating for which parts of the OCT data and/or the image data, in particular which area segments, the neural network (in particular, filter layers of the neural network) responded to most strongly when characterizing the type of ophthalmological inhomogeneity. The activation map is generated, for example by the ophthalmological image processing module 91.

**Fig. 14** shows a flow diagram illustrating a method 500 for adapting a treatment pattern according to the type of optical inhomogeneity in the eye. The method 500 may include steps S1-S3 as described in methods 100, 200, 300, 400. The method 500 includes step S6.

However, in an embodiment, the method includes only step S6 and instead includes an optional step of retrieving, from memory or from a separate device, the type of optical inhomogeneity characterized in the eye. In particular, one of the methods 100, 200, 300, 400 (which includes steps S1-S3) may be performed by an ophthalmological imaging system 1. The results of one of these methods, including the OCT data and/or image data, may be stored in the memory 12 for later retrieval to a laser treatment device 3, or for transmission to a laser treatment device 3.

In step S6, the treatment pattern is adapted. In particular, the treatment pattern is adapted by adjust one or more of the treatment points of the treatment pattern. The treatment points are defined by their location and laser beam parameters, and adapting the treatment pattern comprises adjusting the location or the laser beam parameters, depending on the cataract grade, the OCT data, and/or the image data.

For example, in the case that the optical inhomogeneity in the eye includes a cataract, the treatment points which coincide with one or more area segments and/or volume segments associated with the cataract are adjusted according to the degree of opacity and/or density of the area segments and/or volume segments. For example, the laser power at a particular treatment point, which treatment point coincides with a cataract, may be increased to ensure that the photodisruption threshold is still met. This compensates for the opacification leading to light scattering and therefore loss of energy in the cataract.

For example, if a severe posterior subcapsular cataract is detected, the treatment pattern is adjusted to avoid cutting into the cataractous tissue. The risk of posterior capsule rupture can thus be decreased. In another example, if a severe posterior subcapsular cataract is detected, the treatment pattern should be adjusted to avoid cutting into the cataractous tissue near the posterior capsule, which may be fused with the capsule, which may also be less elastic than normal. Thus a risk of damaging the posterior capsule is averted.

This reduces the time required per patient by minimizing the need for the eye treatment professional (e.g., an ophthalmologist) to adjust the treatment pattern during the procedure in response to observed lens opacity or lens density. Preferably, the method is configured to suggest the adaptation to the treatment pattern, such that the surgeon with must review the suggested adjustment and confirm the exact patent pattern to be used.

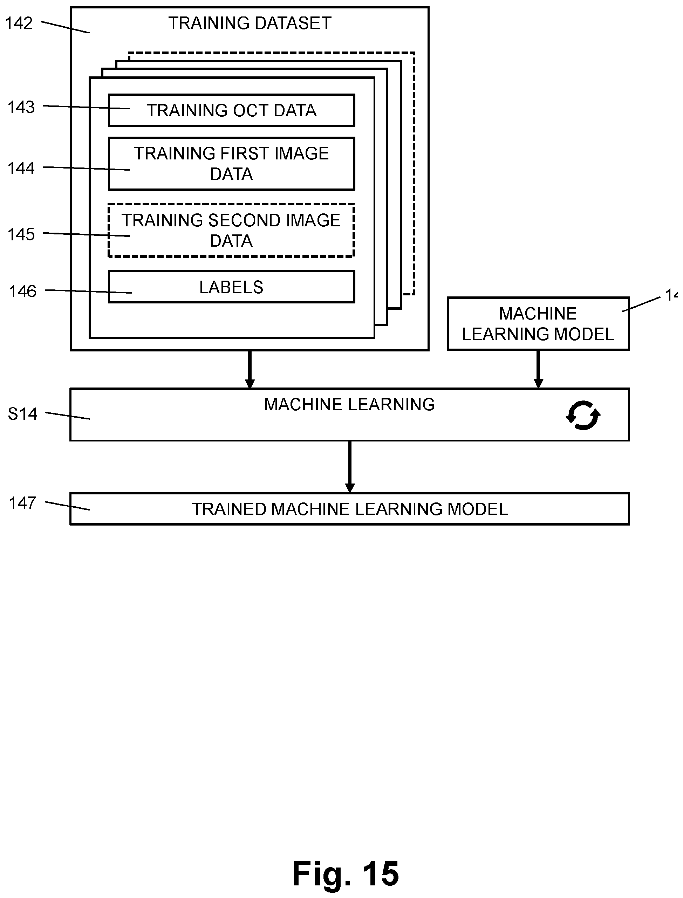

**Fig. 15** shows a diagram indicating a process of training one or more machine learning models 141. The machine learning model(s) 141 may be implemented, for example, as a decision tree, support vector machine, and/or a neural network, as described herein. The machine learning model(s) 141 are trained using supervised learning, in particular using a training dataset 142.

The training dataset 142 comprises data from a plurality of eyes. In particular, the training dataset 142 comprises, for each eye, training OCT data 143, training image data 144, 145, and a label 146 indicating which (if any) type(s) of optical inhomogeneity are present in the eye. The training dataset 142 includes many examples of eyes having all types of optical inhomogeneity, in particular including cataracts, floaters, or opacification of the cornea. In particular for cataracts, many different cataract types, cataract grades, and cataract severity levels are included, and the labels include details about the specific type of optical inhomogeneity. Further, the training dataset 142 may include examples of eyes having no optical inhomogeneity.

Data augmentation techniques may be employed to increase the size of the training dataset 152, in particular by increasing a number of training reference data 143 and training image data 144 from a more limited initial number. Data augmentation can be achieved for example by producing mirrored and/or rotated copies of the limited initial number, random cropping, adding of noise (e.g., speckle noise, gaussian noise, image artifacts), vertical shadowing of image parts as well as local or global brightness adjustments.

In particular, the data augmentation techniques can include, in the training OCT data 143, specific image artifacts known to occur in OCT data. The training image data 144 can similarly include, in the training image data 144, specific image artifacts known to occur in image data. Preferably, the training OCT data 142 is supplemented with the altered OCT data, leaving the initial training OCT data 142 and the initial unchanged. Similar considerations apply regarding the image data 144.

In an embodiment, the training dataset 142 further comprises supplementary patient information including an age of the patient, a sex of the patient, a visual acuity of the eye of the patient, one or more symptoms of the patient, or a medical history of the patient.

The label 146 may additionally include labelled features in the training OCT data 142 and/or the training image data 144, 145. The label 146 may include one or more defined area segments and/or one or more defined volume segments.

The image data 144, 145 relates to directly illuminated images of the eye, retro-illuminated images of the eye, and/or Scheimpflug images. Thereby, the image data 144, 145 may comprise first image data 144, 145 related to a first one of these techniques and optionally second image data 144, 145 related to a second one of these techniques.

In step S14, machine learning is performed during which the machine learning model 141 is iteratively trained to correctly identify the labelled optical inhomogeneity types in the training dataset 142. Thereby, a trained machine learning model 147 is obtained for use in the ophthalmological image processing module 91. Depending on the embodiment, the trained machine learning model 147 may implement the entire ophthalmological image processing module 91 or one or more modules thereof. In particular, where several modules use a trained machine learning model 147, the trained machine learning model 147 will typically not be the same model for each module. For example, while the OCT module 911 and the one or more image modules 912, 913, 914 may use a neural network for image segmentation and/or classification, the synthesis module 915 may use a decision tree.

Depending on the type of machine learning module used, the training phase of step S14 may include different parameters, hyper parameters, or training techniques.

For example, in the case of the machine learning model including a decision tree, a gradient boosting algorithm may be used for optimal machine learning.

In an embodiment, the machine learning module comprises a support vector machine to characterize the optical inhomogeneity. The support vector machine uses the training dataset 142 to generate an optimal hyperplane which is used to characterize the optical inhomogeneity of the eye in question.

The above-described embodiments of the disclosure are exemplary and the person skilled in the art knows that at least some of the components and/or steps described in the embodiments above may be rearranged, omitted, or introduced into other embodiments without deviating from the scope of the present disclosure.

## Claims

1. A computer-implemented method for characterizing an optical inhomogeneity in a human eye (21), the method comprising:
receiving (S1) optical coherence tomography data (92) of the eye (21);
receiving (S2) image data (93) of an image of the eye (21) recorded by a camera (16), the image recorded using one or more of the following imaging techniques: direct illumination of the eye (21), retro illumination of the eye (21), or Scheimpflug imaging; and
characterizing (S3) the optical inhomogeneity as one or more of the following optical inhomogeneity types: a cataract (941), a floater (942), or an opacification of the cornea (943) using the optical coherence tomography data (92) and the image data (93).

2. The method according to claim 1, wherein characterizing (S3) the optical inhomogeneity type further includes characterizing a cataract grade of the cataract (941).

3. The method according to claim 2, wherein the cataract grade includes a cataract type comprising one or more of: a nuclear sclerotic cataract, a cortical spoking cataract, or a posterior subcapsular cataract; optionally the cataract grade includes a cataract severity level associated with at least one of the one or more cataract types.

4. The method according to one of claims 1 to 3, wherein the optical coherence tomography data (92) includes one or more B-scans (922), preferably two or more B-scans arranged plane parallel to each other, or two or more B-scans rotated radially with respect to each other about an optical axis.

5. The method according to one of claims 1 to 4, wherein characterizing (S3) the optical inhomogeneity types comprises:
identifying (S31) one or more area segments of opacity in the eye (21) using one or more of: the optical coherence tomography data (92) or the image data (93), preferably including, for at least one area segment, one or more of: a location in the eye, a two-dimensional size, a two-dimensional shape, a degree of opacity, or a distribution of opacity, and
characterizing (S33) the optical inhomogeneity using the one or more area segments.

6. The method according to one of claims 1 to 5, wherein characterizing (S3) the optical inhomogeneity types comprises:
generating (S34) a three dimensional representation of at least part of the eye (21) using one or more of: the optical coherence tomography data (92) or the image data (93),
identifying (S35) one or more volume segments of opacity in the eye using the three dimensional representation, preferably including, for at least one volume segment, a location in the eye, a three-dimensional size, a three-dimensional shape, a degree of opacity, or a distribution of opacity, and characterizing (S37), in the processor, the optical inhomogeneity using the one or more volume segments.

7. The method according to one of claims 1 to 6, wherein characterizing (S3) the optical inhomogeneity type comprises using a decision tree.

8. The method according to one of claims 1 to 7, wherein characterizing (S3) the optical inhomogeneity type includes using a machine learning model.

9. The method according to claim 8, wherein the machine learning model receives, as an input, the optical coherence tomography data (92) and the image data (93) and generates, as an output, the optical inhomogeneity type (94).

10. The method according to one of claims 8 or 9, wherein the machine learning model comprises a convolutional neural network.

11. The method according to one of claims 8 to 10, wherein the machine learning model is a machine learning model trained using supervised learning and a training dataset (142) comprising optical coherence tomography data (143), image data (144, 145), and a labelled optical inhomogeneity type (146) of a plurality of patients.

12. The method according to one of claims 1 to 11, wherein the method further comprises displaying (S4), on a display (14), an indicator of the optical inhomogeneity type and optionally one or more of: the optical coherence tomography data (92) or the image data (93).

13. The method according to claims 12 and 5, wherein the method further comprises displaying (S5), on the display (14), the one or more area segments overlaid on one or more of: the optical coherence tomography data or the image data.

14. The method according to one of claims 12 or 13, wherein the method further comprises generating one or more activation maps of the neural network, and displaying, on the display (14), the one or more activation maps, preferably overlaid on one or more of: the optical coherence tomography data (92) or the image data (93).

15. The method according to one of claims 1 to 14, wherein the method further comprises adapting (S6) a treatment pattern for laser treatment of the eye (21) using one or more of: the optical inhomogeneity type, the optical coherence tomography data, or the image data.

16. The method according to claim 15, wherein the treatment pattern comprises a sequence of treatment points, each treatment point having associated therewith a location and laser beam parameters, and adapting the treatment pattern comprises adjusting one or more of: the location or the laser beam parameters, for one or more treatment points, depending on one or more of: the cataract grade, the optical coherence tomography data, or the image data.

17. The method according to one of claims 1 to 16, wherein the method further comprises receiving supplementary patient information including one or more of the following: an age of the patient (2), a sex of the patient (2), a visual acuity of the eye of the patient (2), one or more symptoms of the patient (2), or a medical history of the patient (2), and wherein characterizing the cataract grade further comprises using the supplementary patient information.

18. An ophthalmological imaging system (1) comprising a processor (11), an optical coherence tomography system (15), a camera (16), and a lighting system (17), wherein the processor (11) is configured to perform the method according to one of claims 1 to 17.

19. A laser treatment device (3) comprising a base station (31) having a laser source (311) configured to generate a laser beam, an arm (32) connected to the base station (31) configured to provide a beam path for the laser beam, an application head (33) configured to direct the laser beam into an eye (21) of a patient (3), and an ophthalmological imaging system (1) according to claim 18.

20. A computer program product for characterizing a cataract in a human eye (21), comprising computer program code configured to control a processor (11) such that the processor (11) performs the method according to one of claims 1 to 17.

## Patentansprüche

1. Ein computerimplementiertes Verfahren zur Charakterisierung einer optischen Inhomogenität im menschlichen Auge (21), wobei das Verfahren umfasst:
Empfangen (S1) von optischen Kohärenztomographiedaten (92) des Auges (21);
Empfangen (S2) von Bilddaten (93) eines Bildes des Auges (21), das von einer Kamera (16) aufgenommen wurde, wobei das Bild unter Verwendung einer oder mehrerer der folgenden Bildgebungstechniken aufgenommen wurde: direkte Beleuchtung des Auges (21), Rückbeleuchtung des Auges (21) oder Scheimpflug-Bildgebung; und
Charakterisieren (S3) der optischen Inhomogenität als einen oder mehrere der folgenden optischen Inhomogenitätstypen: eine Katarakt (941), eine Glaskörpertrübung (942) oder eine Opazifikation der Hornhaut (943) unter Verwendung der optischen Kohärenztomographiedaten (92) und der Bilddaten (93).

2. Verfahren nach Anspruch 1, wobei das Charakterisieren (S3) des optischen Inhomogenitätstyp ferner das Charakterisieren eines Kataraktgrades der Katarakt (941) umfasst.

3. Verfahren nach Anspruch 2, wobei der Kataraktgrad eine Kataraktart umfasst, der einen oder mehrere der folgenden umfasst: eine Kernkatarakt, eine speichenartige Rindenkatarakt oder eine hintere subkapsuläre Katarakt; optional umfasst der Kataraktgrad einen Kataraktschweregrad, der mit mindestens einem der einen oder mehreren Kataraktarten assoziiert ist.

4. Verfahren nach einem der Ansprüche 1 bis 3, wobei die optischen Kohärenztomographiedaten (92) einen oder mehrere B-Scans (922) umfassen, vorzugsweise zwei oder mehr B-Scans, die ebenparallel zueinander angeordnet sind, oder zwei oder mehr B-Scans, die radial zueinander um eine optische Achse gedreht sind.

5. Verfahren nach einem der Ansprüche 1 bis 4, wobei das Charakterisieren (S3) der optischen Inhomogenitätstypen umfasst:
Identifizieren (S31) eines oder mehrerer Bereichssegmente der Opazität im Auge (21) unter Verwendung eines oder mehrerer der folgenden: der optischen Kohärenztomographiedaten (92) oder der Bilddaten (93), vorzugsweise einschließlich, für mindestens ein Bereichssegment, eines oder mehrerer der folgenden: einer Position im Auge, einer zweidimensionalen Größe, einer zweidimensionalen Form, eines Opazitätsgrads oder einer Opazitätsverteilung, und
Charakterisieren (S33) der optischen Inhomogenität unter Verwendung des einen oder der mehreren Bereichssegmente.

6. Verfahren nach einem der Ansprüche 1 bis 5, wobei das Charakterisieren (S3) der optischen Inhomogenitätstypen umfasst:
Erzeugen (S34) einer dreidimensionalen Darstellung mindestens eines Teils des Auges (21) unter Verwendung eines oder mehrerer der folgenden: der optischen Kohärenztomographiedaten (92) oder der Bilddaten (93),
Identifizieren (S35) eines oder mehrerer Volumensegmente der Opazität im Auge unter Verwendung der dreidimensionalen Darstellung, vorzugsweise einschließlich, für mindestens ein Volumensegment, einer Position im Auge, einer dreidimensionalen Größe, einer dreidimensionalen Form, eines Opazitätsgrads oder einer Opazitätsverteilung, und
Charakterisieren (S37) der optischen Inhomogenität im Prozessor unter Verwendung des einen oder der mehreren Volumensegmente.

7. Verfahren nach einem der Ansprüche 1 bis 6, wobei das Charakterisieren (S3) des optischen Inhomogenitättyps die Verwendung eines Entscheidungsbaums umfasst.

8. Verfahren nach einem der Ansprüche 1 bis 7, wobei das Charakterisieren (S3) des optischen Inhomogenitätstyps die Verwendung eines maschinellen Lernmodells umfasst.

9. Verfahren nach Anspruch 8, wobei das maschinelle Lernmodell als Eingabe die optischen Kohärenztomographiedaten (92) und die Bilddaten (93) empfängt und als Ausgabe den optischen Inhomogenitätstyp (94) erzeugt.

10. Verfahren nach einem der Ansprüche 8 oder 9, wobei das maschinelle Lernmodell ein faltendes neuronales Netzwerk umfasst.

11. Verfahren nach einem der Ansprüche 8 bis 10, wobei das maschinelle Lernmodell ein maschinelles Lernmodell ist, das unter Verwendung von überwachtem Lernen und einem Trainingsdatensatz (142) trainiert wurde, der optische Kohärenztomographiedaten (143), Bilddaten (144, 145) und einen gekennzeichneten optischen Inhomogenitätstyp (146) einer Vielzahl von Patienten umfasst.

12. Verfahren nach einem der Ansprüche 1 bis 11, wobei das Verfahren ferner das Anzeigen (S4) eines Indikators für den optischen Inhomogenitätstyp auf einer Anzeige (14) umfasst und optional eines oder mehrerer der folgenden: die optischen Kohärenztomographiedaten (92) oder die Bilddaten (93).

13. Verfahren nach den Ansprüchen 12 und 5, wobei das Verfahren ferner das Anzeigen (S5) eines oder mehrerer Bereichssegmente auf der Anzeige (14) umfasst, die über eine oder mehrere der folgenden gelegt sind: die optischen Kohärenztomographiedaten oder die Bilddaten.

14. Verfahren nach einem der Ansprüche 12 oder 13, wobei das Verfahren ferner das Erzeugen einer oder mehrerer Aktivierungskarten des neuronalen Netzwerks und das Anzeigen der einen oder mehreren Aktivierungskarten auf der Anzeige (14) umfasst, vorzugsweise überlagert auf einer oder mehreren der folgenden: der optischen Kohärenztomographiedaten (92) oder den Bilddaten (93).

15. Verfahren nach einem der Ansprüche 1 bis 14, wobei das Verfahren ferner das Anpassen (S6) eines Behandlungsmusters für die Laserbehandlung des Auges (21) unter Verwendung eines oder mehrerer der folgenden umfasst: den optischen Inhomogenitätstyp, die optischen Kohärenztomographiedaten oder die Bilddaten.

16. Verfahren nach Anspruch 15, wobei das Behandlungsmuster eine Folge von Behandlungspunkten umfasst, wobei jedem Behandlungspunkt ein Ort und Laserstrahlparameter zugeordnet sind, und das Anpassen des Behandlungsmusters das Einstellen eines oder mehrerer der folgenden umfasst: den Ort oder die Laserstrahlparameter für einen oder mehrere Behandlungspunkte, abhängig von einem oder mehreren der folgenden: dem Kataraktgrad, den optischen Kohärenztomographiedaten oder den Bilddaten.

17. Verfahren nach einem der Ansprüche 1 bis 16, wobei das Verfahren ferner das Empfangen von ergänzenden Patienteninformationen umfasst, die eines oder mehrere der folgenden umfassen: ein Alter des Patienten (2), ein Geschlecht des Patienten (2), eine Sehschärfe des Auges des Patienten (2), ein oder mehrere Symptome des Patienten (2) oder eine Krankengeschichte des Patienten (2), und wobei das Charakterisieren des Kataraktgrades ferner das Verwenden der zusätzlichen Patienteninformationen umfasst.

18. Ein ophthalmologisches Bildgebungssystem (1), das einen Prozessor (11), ein optisches Kohärenztomographiesystem (15), eine Kamera (16) und ein Beleuchtungssystem (17) umfasst, wobei der Prozessor (11) so konfiguriert ist, dass er das Verfahren gemäß einem der Ansprüche 1 bis 17 ausführt.

19. Laserbehandlungsvorrichtung (3) mit einer Basisstation (31) mit einer Laserquelle (311), die so konfiguriert ist, dass sie einen Laserstrahl erzeugt, einem Arm (32), der mit der Basisstation (31) verbunden ist und so konfiguriert ist, dass er einen Strahlengang für den Laserstrahl bereitstellt, einem Anwendungskopf (33), der so konfiguriert ist, dass er den Laserstrahl in ein Auge (21) eines Patienten (3) lenkt, und ein ophthalmologisches Bildgebungssystem (1) gemäß Anspruch 18.

20. Ein Computerprogrammprodukt zur Charakterisierung einer Katarakt in einem menschlichen Auge (21), umfassend einen Computerprogrammcode, der so konfiguriert ist, dass er einen Prozessor (11) so steuert, dass der Prozessor (11) das Verfahren gemäß einem der Ansprüche 1 bis 17 ausführt.

## Revendications

1. Procédé mis en œuvre par ordinateur pour caractériser une inhomogénéité optique dans un œil humain (21), le procédé comprenant :
la réception (S1) de données de tomographie par cohérence optique (92) de l'œil (21) ;
la réception (S2) de données d'image (93) d'une image de l'œil (21) enregistrée par une caméra (16), l'image étant enregistrée à l'aide d'une ou plusieurs des techniques d'imagerie suivantes : illumination directe de l'œil (21), rétro-illumination de l'œil (21) ou imagerie Scheimpflug ; et
caractériser (S3) l'inhomogénéité optique comme un ou plusieurs des types d'inhomogénéité optique suivants : une cataracte (941), des mouches volantes (942) ou une opacification de la cornée (943) à l'aide des données de tomographie par cohérence optique (92) et des données d'image (93).

2. Procédé selon la revendication 1, dans lequel la caractérisation (S3) du type d'inhomogénéité optique comprend en outre la caractérisation d'un degré de cataracte de la cataracte (941).

3. Procédé selon la revendication 2, dans lequel le degré de cataracte comprend un type de cataracte comprenant un ou plusieurs des suivants : une cataracte nucléaire sclérotique, une cataracte corticale ou une cataracte sous-capsulaire postérieure ; en option, le degré de cataracte comprend un niveau de gravité de la cataracte associé à au moins un des un ou plusieurs types de cataracte.

4. Procédé selon l'une des revendications 1 à 3, dans lequel les données de tomographie par cohérence optique (92) comprennent un ou plusieurs balayages B (922), de préférence deux ou plusieurs balayages B disposés dans un plan parallèle les uns aux autres, ou deux ou plusieurs balayages B tournés radialement les uns par rapport aux autres autour d'un axe optique.

5. Procédé selon l'une des revendications 1 à **4,** dans lequel la caractérisation (S3) des types d'inhomogénéité optique comprend :
l'identification (S31) d'un ou plusieurs segments de zone d'opacité dans l'œil (21) à l'aide d'un ou plusieurs des suivants : les données de tomographie par cohérence optique (92) ou les données d'image (93), comprenant de préférence, pour au moins un segment de zone, un ou plusieurs des suivants : un emplacement dans l'œil, une taille bidimensionnelle, une forme bidimensionnelle, un degré d'opacité ou une distribution d'opacité, et
caractériser (S33) l'inhomogénéité optique à l'aide d'un ou plusieurs segments de zone.

6. Procédé selon l'une des revendications 1 à 5, dans lequel la caractérisation (S3) des types d'inhomogénéité optique comprend :
générer (S34) une représentation tridimensionnelle d'au moins une partie de l'œil (21) en utilisant un ou plusieurs des suivants : les données de tomographie par cohérence optique (92) ou les données d'image (93),
identifier (S35) un ou plusieurs segments de volume d'opacité dans l'œil à l'aide de la représentation tridimensionnelle, comprenant de préférence, pour au moins un segment de volume, un emplacement dans l'œil, une taille tridimensionnelle, une forme tridimensionnelle, un degré d'opacité ou une distribution d'opacité, et
caractériser (S37), dans le processeur, l'inhomogénéité optique à l'aide d'un ou plusieurs segments de volume.

7. Procédé selon l'une des revendications 1 à 6, dans lequel la caractérisation (S3) du type d'inhomogénéité optique comprend l'utilisation d'un arbre de décision.

8. Procédé selon l'une des revendications 1 à 7, dans lequel la caractérisation (S3) du type d'inhomogénéité optique comprend l'utilisation d'un modèle d'apprentissage automatique.

9. Procédé selon la revendication 8, dans lequel le modèle d'apprentissage automatique reçoit, en entrée, les données de tomographie par cohérence optique (92) et les données d'image (93) et génère, en sortie, le type d'inhomogénéité optique (94).

10. Procédé selon l'une des revendications 8 ou 9, dans lequel le modèle d'apprentissage automatique comprend un réseau neuronal convolutif.

11. Procédé selon l'une des revendications 8 à 10, dans lequel le modèle d'apprentissage automatique est un modèle d'apprentissage automatique entraîné à l'aide d'un apprentissage supervisé et d'un ensemble de données d'entraînement (142) comprenant des données de tomographie par cohérence optique (143), des données d'image (144, 145) et un type d'inhomogénéité optique étiqueté (146) d'une pluralité de patients.

12. Procédé selon l'une des revendications 1 à 11, dans lequel le procédé comprend en outre l'affichage (S4), sur un écran (14), d'un indicateur du type d'inhomogénéité optique et, éventuellement, d'un ou plusieurs des suivants : les données de tomographie par cohérence optique (92) ou les données d'image (93).

13. Procédé selon les revendications 12 et 5, dans lequel le procédé comprend en outre l'affichage (S5), sur l'écran (14), d'un ou plusieurs segments de zone superposés sur l'un ou plusieurs des suivants : les données de tomographie par cohérence optique ou les données d'image.

14. Procédé selon l'une des revendications 12 ou 13, dans lequel le procédé comprend en outre la génération d'une ou plusieurs cartes d'activation du réseau neuronal, et l'affichage, sur l'écran (14), de la ou des cartes d'activation, de préférence superposées sur l'un ou plusieurs des suivants : les données de tomographie par cohérence optique (92) ou les données d'image (93).

15. Procédé selon l'une des revendications 1 à 14, dans lequel le procédé comprend en outre l'adaptation (S6) d'un modèle de traitement pour le traitement au laser de l'œil (21) en utilisant un ou plusieurs des suivants : le type d'inhomogénéité optique, les données de tomographie par cohérence optique ou les données d'image.

16. Procédé selon la revendication 15, dans lequel le modèle de traitement comprend une séquence de points de traitement, chaque point de traitement étant associé à un emplacement et à des paramètres de faisceau laser, et l'adaptation du modèle de traitement comprend l'ajustement d'un ou plusieurs des suivants : l'emplacement ou les paramètres de faisceau laser, pour un ou plusieurs points de traitement, en fonction d'un ou plusieurs des suivants : le degré de cataracte, les données de tomographie par cohérence optique ou les données d'image.

17. Procédé selon l'une des revendications 1 à 16, dans lequel le procédé comprend en outre la réception d'informations supplémentaires sur le patient, comprenant un ou plusieurs des suivants : l'âge du patient (2), le sexe du patient (2), l'acuité visuelle de l'œil du patient (2), un ou plusieurs symptômes du patient (2) ou les antécédents médicaux du patient (2), et dans lequel la caractérisation du degré de cataracte comprend en outre l'utilisation des informations supplémentaires sur le patient.

18. Système d'imagerie ophtalmologique (1) comprenant un processeur (11), un système de tomographie par cohérence optique (15), une caméra (16) et un système d'éclairage (17), dans lequel le processeur (11) est configuré pour exécuter le procédé selon l'une des revendications 1 à 17.

19. Dispositif de traitement au laser (3) comprenant une station de base (31) comportant une source laser (311) configurée pour générer un faisceau laser, un bras (32) relié à la station de base (31) configuré pour fournir un trajet de faisceau pour le faisceau laser, une tête d'application (33) configurée pour diriger le faisceau laser dans un œil (21) d'un patient (3), et un système d'imagerie ophtalmologique (1) selon la revendication 18.

20. Produit logiciel destiné à caractériser une cataracte dans un œil humain (21), comprenant un code logiciel configuré pour commander un processeur (11) de telle sorte que le processeur (11) exécute le procédé selon l'une des revendications 1 à 17.
